(19) Europäisches Patentamt
European Patent Office
Office européen des brevets

(11) **EP 3 765 589 B1**

(12) **EUROPÄISCHE PATENTSCHRIFT**

(45) Veröffentlichungstag und Bekanntmachung des Hinweises auf die Patenterteilung:
**23.02.2022 Patentblatt 2022/08**

(21) Anmeldenummer: **19709739.7**

(22) Anmeldetag: **14.03.2019**

(51) Internationale Patentklassifikation (IPC):
**C12M 1/00** (2006.01)  **C12M 1/34** (2006.01)
**G01F 23/24** (2006.01)

(52) Gemeinsame Patentklassifikation (CPC):
**C12M 41/44; C12M 41/14; G01F 23/246**

(86) Internationale Anmeldenummer:
**PCT/EP2019/056447**

(87) Internationale Veröffentlichungsnummer:
**WO 2019/175325 (19.09.2019 Gazette 2019/38)**

(54) **FÜLLSTANDSMESSVORRICHTUNG FÜR EINE LABORSCHRANKVORRICHTUNG**

LEVEL MEASURING APPARATUS FOR A LABORATORY CABINET DEVICE

DISPOSITIF DE MESURE DE NIVEAU DE REMPLISSAGE POUR UN DISPOSITIF ARMOIRE DE LABORATOIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2018 EP 18162387**

(43) Veröffentlichungstag der Anmeldung:
**20.01.2021 Patentblatt 2021/03**

(73) Patentinhaber: **Eppendorf SE**
**22339 Hamburg (DE)**

(72) Erfinder:
• **GRAFF, Andreas**
**22339 Hamburg (DE)**
• **STRANZINGER, Martin**
**22339 Hamburg (DE)**

(74) Vertreter: **Kirchner, Christian**
**Wallinger Ricker Schlotter Tostmann**
**Patent- und Rechtsanwälte Partnerschaft mbB**
**Zweibrückenstrasse 5 - 7**
**80331 München (DE)**

(56) Entgegenhaltungen:
**EP-A1- 2 067 849   JP-A- 2002 188 977**

**Beschreibung**

Die Erfindung betrifft eine Füllstandsmessvorrichtung für eine Laborschrankvorrichtung, zur Messung eines Füllstands in einem Flüssigkeitsbehälter im Innenraum der Laborschrankvorrichtung. Die Laborschrankvorrichtung dient dem Lagern von Laborproben und ist insbesondere ein Temperierschrank für das Temperieren von Laborproben, insbesondere einen Inkubator für das Wachstum von Zellkulturen.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen $CO_2$-Inkubatoren. Die Atmosphäre wird durch Luft mit einem bestimmten $CO_2$- und $O_2$-Gehalt und einer bestimmten Luftfeuchtigkeit gebildet, eine geeignete Temperatur ist oftmals 37 °C. Solche Temperierschränke weisen ein Gehäuse auf, beispielsweise ein Außengehäuse, mit einer Gehäuseöffnung, durch die der Benutzer die Proben im Gehäuseinneren lagert und wieder entnimmt. Dabei entweicht regelmäßig auch Wasserdampf aus dem Innenraum. Die Inkubatoren beinhalten deshalb einen nach oben geöffneten, mit Wasser gefüllten Flüssigkeitsbehälter, mittels dem im Innenraum durch Verdunsten des Wassers eine relative Luftfeuchtigkeit von größer als 90% hergestellt wird. Um den Wasserverbrauch zu erfassen und einer Komplettleerung des Wasserbehälters zuvor zu kommen, weisen solche Temperierschränke oftmals Füllstandsmesseinrichtungen auf.

Aus der EP 2 067 849 B1 ist ein Inkubator mit zwei NTC-Sensoren bekannt, die beide im Wasserbad des Inkubators auf einem Messniveau angeordnet sind, die mit unterschiedlichen Temperaturen betrieben werden und deren Ausgangsspannungen ausgewertet werden, um das Unterschreiten des Messniveaus zu erfassen. Aus dem Dokument JP2002188977 A ist eine Füllstandsmessvorrichtung entsprechend dem Oberbegriff des Anspruchs 1 bekannt.

Der vorliegenden Erfindung liegt die Aufgabe zugrunde, eine zuverlässige und effizient gestaltete Füllstandsmessvorrichtung für eine Laborschrankvorrichtung, insbesondere einen Temperierschrank, bereitzustellen.

Die Erfindung löst diese Aufgabe durch die Füllstandsmessvorrichtung gemäß Anspruch 1. Bevorzugte Ausgestaltungen sind insbesondere Gegenstände der Unteransprüche.

Die erfindungsgemäß gestaltete Füllstandsmessvorrichtung weist NTC-(englisch: "negative temperature coefficient")-Temperatursensoren auf, die auch als NTC-Thermistoren bekannt sind. Solche Temperatursensoren sind selbstregulierend. Sie werden mit einem Strom versorgt, der so groß ist, dass es zu einer Selbsterwärmung kommt. Durch den Strom werden sie anfangs erwärmt und auf einer Übertemperatur gehalten, die über der Temperatur ihrer Umgebung liegt. Durch das NTC-Verhalten, gemäß dem der elektrische Widerstand mit der -durch die Wärmeabgabe an die Umgebung bedingten- sinkenden Temperatur steigt, begrenzt sich die Leistung selber und es stellt sich ein stationärer Gleichgewichtszustand ein. Der erste NTC-Temperatursensor wird so angeordnet, dass er nie mit der Flüssigkeit in Berührung kommt, der zweite NTC-Temperatursensor ist im Normalfall in der Flüssigkeit und ändert seine Wärmeabgabeleistung, sobald der Flüssigkeitspegel unter den zweiten NTC-Temperatursensor sinkt. Der erste NTC-Temperatursensor dient deshalb als Referenzsensor, der seine Referenz-Funktion unabhängig von der Temperatur des Innenraums der Laborschrankvorrichtung erfüllt, so dass ein besonders zuverlässiger Betrieb der mit einfachen Mitteln realisierten Füllstandsmessvorrichtung gewährleistet ist.

Der erste NTC-Temperatursensor und der zweite NTC-Temperatursensor geben eine unterschiedliche Wärmeleistung an die jeweilige Umgebung ab, wenn diese Umgebungen unterschiedliche Wärmeleitfähigkeiten aufweisen. Da die Wärmeleitfähigkeit von Luft wesentlich schlechter ist als die einer Flüssigkeit, insbesondere Wasser, gibt der zweite NTC-Temperatursensor eine höhere Wärmeleistung ab als der erste NTC-Temperatursensor. Dadurch stellt sich am zweiten NTC-Temperatursensor ein höherer elektrischer Widerstand ein als am ersten NTC-Temperatursensor, so dass die Spannung über den zweiten NTC-Temperatursensor stärker abfällt als am erste NTC-Temperatursensor, solange der zweite NTC-Temperatursensor die Flüssigkeit kontaktiert. Liegt der zweite NTC-Temperatursensor bei gesunkenem Füllstand dann in derselben Umgebung (z.B. Luft) wie der erste NTC-Temperatursensor, ändert sich der Widerstand des zweiten NTC-Temperatursensors bzw. gleicht sich dem Widerstand des ersten NTC-Temperatursensors an, und diese Änderung lässt sich messen. Insbesondere ist der Betrag der Differenz der Ausgangsspannungen des ersten und zweiten NTC-Temperatursensors im ersten Zustand größer, wenn sich der zweite NTC-Temperatursensor bei normalem Füllstand in Flüssigkeit befindet und der erste in Luft, als im zweiten Zustand, wenn sich beide NTC-Temperatursensoren in Luft befinden.

Der erste und zweite NTC-Temperatursensor sind vorzugsweise so ausgewählt, dass sie mit einer niedrigen Spannung von vorzugsweise 15 bis 35 Volt, vorzugsweise 20 bis 30 Volt betreibbar sind. Vorzugsweise werden deshalb niederohmige NTC-Temperatursensoren mit 1-2 kOhm verwendet. Sie sind vorzugsweise so ausgewählt, dass sie Umgebungstemperaturen von bis zu 200° C, vorzugsweise bis zu 190° C und vorzugsweise bis zu 180° C im bestimmungsgemäßen Betrieb tolerieren bzw. diese Temperaturen unbeschadet überstehen. Dadurch können solche Temperatursensoren im Innenraum einer Laborschrankvorrichtung, insbesondere eines Temperierschrank bzw. Inkubators verbleiben, wenn dieser auf die genannte hohe Temperatur erhitzt wird, beispielsweise zum Zweck der Sterilisation des Innenraums bei 180 °C. Die Verwendung der mit einer solchen Füllstandsmessvorrichtung versehenen Laborschrankvorrichtung ist deshalb besonders effizient, da die Füllstandsmessvorrichtung nicht vor einer Sterilisation entfernt werden muss. Ein geeigneter NTC-Temperatursensor ist beispielsweise der G2K3348 Radial Glass Thermistor von Measurement Specialties, Hampton, Virginia, USA.

Die elektronische Messeinrichtung weist vorzugsweise elektronische Schaltkreise auf. Die elektronische Messeinrichtung ist vorzugsweise dazu eingerichtet, einen ersten Vergleichswert zu ermitteln, wenn - bzw. solange - der Füllstand im Flüssigkeitsbehälter oberhalb der Position des zweiten NTC-Temperatursensors steht, und einen vom ersten Vergleichswert abweichenden zweiten Vergleichswert zu erfassen, wenn der Füllstand im Flüssigkeitsbehälter unter die Position den zweiten NTC-Temperatursensor gesunken ist. Der erste und zweite Vergleichswert werden insbesondere ermittelt, indem der Vergleichswert, insbesondere ein Differenzwert, der ersten und zweiten elektrischen Größe kontinuierlich erfasst wird und dann mit einem Referenzwert verglichen wird, um die Entscheidung zu treffen, ob ein erster Vergleichswert vorliegt (erster Zustand bzw. Normalzustand) oder ein zweiter Vergleichswert vorliegt (zweiter Zustand bzw. Fehlerzustand).

Vorzugsweise weist die Füllstandsmessvorrichtung eine Auswertungseinrichtung auf, die insbesondere ein zur Durchführung der betreffenden Auswertung programmierbarer Mikrokontroller oder Computer ist. Die Auswertungseinrichtung kann insbesondere auch einen A/D-Wandler (AD-Converter, ADC) zur Digitalisierung von analogen Messsignalen der elektronischen Messeinrichtung aufweisen. Die Auswertungseinrichtung kann Bestandteil einer elektronischen Steuereinrichtung sein, die die elektrisch steuerbaren Funktionen, insbesondere die Regelung der Innenraumtemperatur, der Laborschrankvorrichtung steuert. Die Auswertungseinrichtung weist vorzugsweise eine Datenspeichereinrichtung zur flüchtigen oder nicht-flüchtigen Speicherung von Daten auf, und/oder vorzugsweise eine Datenverarbeitungseinrichtung, die insbesondere dazu eingerichtet ist, den zweiten Vergleichswert vom ersten Vergleichswert zu unterscheiden, indem der fortlaufend gemessene Vergleichswert mit einem Referenzwert verglichen wird, der in der Datenspeichereinrichtung gespeichert ist. Die Datenspeichereinrichtung und/oder die Datenverarbeitungseinrichtung können Bestandteile einer elektronischen Steuereinrichtung sein, die die elektrisch steuerbaren Funktionen, insbesondere die Regelung der Innenraumtemperatur, der Laborschrankvorrichtung steuert.

Vorzugsweise ist die elektronische Messeinrichtung und/oder die Auswertungseinrichtung dazu eingerichtet, in Abhängigkeit von der Auswertung des Vergleichswerts ein Ausgabesignal und/oder Ausgabedaten zu erzeugen, die über eine Benutzerschnittstelleneinrichtung an den Benutzer ausgegeben wird. Auf diese Weise kann der Benutzer insbesondere über das Absinken des Füllstands unter einen Schwellwert informiert werden. Dieser Schwellwert entspricht dem genannten Referenzwert. Die Benutzerschnittstelleneinrichtung kann insbesondere über ein Display der Füllstandsmessvorrichtung oder der Laborschrankvorrichtung, welche diese Füllstandsmessvorrichtung aufweist, die Information über das Absinken des Füllstands unter einen Schwellwert ausgeben. Die Benutzerschnittstelleneinrichtung kann auch dazu eingerichtet sein, ein akustisches Hinweissignal auszugeben und/oder über eine Datenfernverbindung, zum Beispiel über ein LAN, an das die Laborschrankvorrichtung angeschlossen ist, die Ausgabedaten über das Absinken des Füllstands unter einen Schwellwert versenden.

Die elektronische Messeinrichtung weist vorzugsweise eine elektronische Schaltung auf, die den ersten NTC-Temperatursensor und den zweiten NTC-Temperatursensor beinhaltet. Insbesondere werden die erste und die zweite elektrische Größe analogelektrisch ausgewertet. Es ist aber auch möglich, dass die erste und die zweite elektrische Größe digitalisiert und dann digital ausgewertet werden. Vorzugsweise ist mindestens ein A/D-Konverter vorgesehen, der die erste und die zweite elektrische Größe digitalisiert.

Vorzugsweise ist die elektronische Messeinrichtung so eingerichtet, dass die erste und die zweite elektrische Größe analogelektrisch und/oder ausgewertet werden, indem eine Differenzbildung zwischen der ersten und zweiten elektrischen Größe erfolgt, analog oder digital, und der Vergleichswert auf der Differenz der ersten und zweiten elektrischen Größe beruht bzw. der Vergleichswert den Differenzwert der ersten und zweiten elektrischen Größe beinhaltet, der Vergleichswert also insbesondere die Differenz der ersten und zweiten elektrischen Größe oder die Differenz der zweiten und ersten elektrischen Größe ist. Anstelle der Differenzbildung, oder zusätzlich dazu, kann der Vergleich der ersten und zweiten elektrischen Größe auch eine andere mathematische Operation beinhalten, insbesondere eine Addition, Multiplikation und insbesondere eine Quotientenbildung der ersten und zweiten elektrischen Größe.

Die elektronische Messeinrichtung ist vorzugsweise so eingerichtet, dass der erste und der zweite NTC-Temperatursensor in einer Brückenschaltung angeordnet sind. Eine Brückenschaltung ist insbesondere eine Wheatstonesche Messbrücke. Eine solche Schaltung eignet sich besonders gut zur genauen Auswertung kleiner Widerstandsänderungen bzw. kleiner Spannungsänderungen. Die elektronische Messeinrichtung ist vorzugsweise so eingerichtet, dass als der erste und der zweite Vergleichswert jeweils die Brückenspannung der Brückenschaltung verwendet wird.

Die elektronische Messeinrichtung weist vorzugsweise eine spannungsgesteuerte Stromquelle als elektronische Schaltung auf, deren Eingangsspannung die Brückenspannung ist. Der Ausgangsstrom wird vorzugsweise verwendet, um über einen Lastwiderstand eine massebezogene Ausgangsspannung zu erzeugen, die insbesondere an einen ADC-Eingang geht.

Die Füllstandsmessvorrichtung weist eine Halteeinrichtung zum Halten des ersten und des zweiten NTC-Temperatursensors im Abstand zu einer Innenwand der Laborschrankvorrichtung auf. Der erste und der zweite NTC-Temperatursensor werden vorzugsweise so von der Halteeinrichtung gehalten, dass der erste NTC-Temperatursensor im Normalfall, wenn der Flüssigkeitsbehälter ausreichend mit Flüssigkeit befüllt ist, außerhalb der Flüssigkeit angeordnet ist und der zweite NTC-Temperatursensor im Normalfall innerhalb der Flüssigkeit angeordnet ist, und der erste NTC-Temperatursensor im Fehlerfall, wenn der Flüssigkeitsbehälter nicht ausreichend mit Flüssigkeit befüllt ist, weiterhin außerhalb der Flüssigkeit angeordnet ist, und der zweite NTC-Temperatursensor im Fehlerfall auch außerhalb der Flüssigkeit angeordnet ist.

Die Halteeinrichtung weist einen an einer Innenwand, insbesondere inneren Rückwand, der Laborschrankvorrichtung befestigbaren Haltearm auf, der insbesondere für eine horizontal gerichtete Anordnung vorgesehen ist und der den ersten NTC-Temperatursensor trägt und an dem ein, elastisch verformbarerer, zweiter Haltearm angebracht ist, der für eine vertikal nach unten gerichtete Anordnung vorgesehen ist und der den zweiten NTC-Temperatursensor trägt. Der elastisch verformbarer zweiter Haltearm mit daran angeordnetem zweiten NTC-Temperatursensor bietet den Vorteil, dass dieser Haltearm verbogen werden kann, der zweite NTC-Temperatursensor mithin angehoben werden kann, ohne dass der Haltearm demontiert werden muss. Dadurch kann die Entnahme des Flüssigkeitsbehälters, insbesondere der Wanne, im montierten Zustand der zweiten NTC-Temperatursensoren vereinfacht werden.

Vorzugsweise weist die Halteeinrichtung eine Kapseleinrichtung aus einem ersten Material (M1) auf zur teilweisen oder - im Wesentlichen - vollständigen Verkapselung des ersten und/oder des zweiten NTC-Temperatursensors. Vorzugsweise weist der erste NTC-Temperatursensor mindestens eine - oder genau eine - Sensorkontaktfläche auf, die thermisch mit dem temperaturabhängigen Widerstand des NTC-Temperatursensors gekoppelt ist und die nach außen gerichtet ist, wo sie an die Umgebung des NTC-Temperatursensors grenzt. Der NTC-Temperatursensor und dessen Sensorkontaktfläche sind dabei vorzugsweise von einer Fassung eingehüllt, und sind insbesondere von der Kapseleinrichtung größtenteils eingekapselt. Auch der zweite NTC-Temperatursensor weist vorzugsweise eine Sensorkontaktfläche auf, die als zweite Sensorkontaktfläche bezeichnet wird. Die mindestens eine Sensorkontaktfläche des ersten und/oder des zweiten NTC-Temperatursensors ist aus einem zweiten Material M2 gefertigt. Die Kapseleinrichtung fasst vorzugsweise die mindestens eine Sensorkontaktfläche ein. Vorzugsweise begrenzen die Kapseleinrichtung und die mindestens Sensorkontaktfläche den ersten und/oder den zweiten NTC-Temperatursensor nach außen.

Das zweite Material (M2) weist insbesondere eine höhere Wärmeleitfähigkeit auf als das erste Material (M1). Bei einem als Temperierschrank, insbesondere einem $CO_2$ Inkubator, besteht die Anforderung, dass die genau definierte Temperatur des Innenraums nicht beeinflusst werden soll. Die NTC-Temperatursensoren werden aber bei einer Übertemperatur betrieben. Durch die Kapseleinrichtung aus schlechter wärmeleitendem Material M1 werden die Sensoren nach außen thermisch isoliert. Dadurch wird einerseits eine störende Erwärmung des Innenraums verhindert und andererseits eine ungewollte Abkühlung der NTC-Temperatursensoren verhindert. Der Wärmefluss zwischen Sensor und Innenraum konzentriert sich insbesondere auf den kleinen Bereich der mindestens einen Sensorkontaktfläche. Durch diese Ausgestaltung können NTC-Temperatursensoren mit geringer Leistung verwendet werden.

Das erste Material (M1) ist vorzugsweise ein Kunststoff, insbesondere ein Gummi oder ein Elastomer, das insbesondere Temperaturen von bis zu 200 ° C oder 180 ° C widersteht. Das zweite Material (M2) ist vorzugsweise ein Metall, insbesondere Edelstahl.

Vorzugsweise weisen der erste und/oder zweite NTC-Temperatursensor jeweils eine Fassung aus einem Material M3 auf, das ebenfalls schlechter wärmeleitend ist als das Material M2 und den Sensor weiter thermisch isoliert. Vorzugsweis sind der erste NTC-Temperatursensor und dessen Sensorkontaktfläche und/oder der zweite NTC-Temperatursensor und dessen Sensorkontaktfläche jeweils von einer Fassung eingefasst, so dass vorzugsweise die Kapseleinrichtung, die Fassung des ersten und/oder des zweiten NTC-Temperatursensors und die mindestens eine Sensorkontaktfläche den ersten und/oder den zweiten NTC-Temperatursensor nach außen zum Innenraum der Laborschrankvorrichtung begrenzen. Vorzugsweise ist das dritte Material (M3) Polyetheretherketon (PEEK), wodurch eine ausgezeichnete thermische und chemische Widerstandsfähigkeit erreicht wird.

Vorzugsweise bestehen die Halteeinrichtung, der erste NTC-Temperatursensor und der zweite NTC-Temperatursensor aus Materialien, die Betriebstemperaturen des Innenraums der Laborschrankvorrichtung von bis zu 180° C oder bis zu 200 °C unbeschadet widerstehen.

Die Erfindung betrifft insbesondere auch eine Laborschrankvorrichtung zum Lagern von Laborproben, insbesondere einen Temperierschrank, insbesondere einen Inkubator, mit einem Flüssigkeitsbehälter im Innenraum der Laborschrankvorrichtung und einer erfindungsgemäßen Füllstandsmessvorrichtung nach Anspruch 1 zur Messung eines Füllstands in dem Flüssigkeitsbehälter. Die Füllstandsmessvorrichtung nach Anspruch 1 dient insbesondere der Messung des Füllstands des Flüssigkeitsbehälters einer Luftbefeuchtungseinrichtung eines Inkubators.

Die Laborschrankvorrichtung zum Lagern von Laborproben ist insbesondere ein Temperierschrank zum Temperieren von Laborproben. Solche Geräte werden elektrisch betrieben und weisen einen Spannungsanschluss auf. Über diesen können auch die NTC-Temperatursensoren betrieben werden.

Der Temperierschrank temperiert die Laborproben, das heißt, er hält das Gehäuseinnere und damit die dort lagernden Laborproben im Rahmen von Toleranzen bei einer Zieltemperatur, insbesondere hält er das Gehäuseinnere durch eine Temperaturregelung auf einer insbesondere vom Benutzer einstellbaren Solltemperatur. Diese kann über der Raumtemperatur (Umgebungstemperatur) liegen, wie dies bei einem Wärmeschrank oder Inkubator der Fall ist, oder kann unter der Raumtermperatur liegen, wie dies bei einem Kühlschrank oder Gefrierschrank der Fall ist. Bei einer als Klimaschrank ausgebildeten Laborschrankvorrichtung wird vorzugsweise auch ein im Inneren des Gehäuses vorherrschender Klimaparameter im Rahmen von Toleranzen geregelt. Dieser Klimaparameter kann die Luftfeuchtigkeit sein, und/oder eine Gaskonzentration, z.B. eine $CO_2$, $N_2$ und/oder $O_2$-Konzentration. Ein solcher Klimaschrank ist beispielsweise ein $CO_2$ Inkubator für aus lebenden Zellkulturen bestehende Laborproben.

Mit Temperierschränken bzw. $CO_2$ Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen $CO_2$-Inkubatoren. Die Atmosphäre wird durch Luft mit einem bestimmten $CO_2$- und $O_2$-Gehalt und einer bestimmten relativen Luftfeuchtigkeit von über 90%, insbesondere 95%, gebildet, eine geeignete Temperatur ist oftmals 37 °C, dieser kann aber je nach den Anforderungen der Laborproben vom Benutzer eingestellt werden.

Das Gehäuse der Laborschrankvorrichtung ist vorzugsweise ein äußeres Gehäuse, dessen Gehäusewände mit der Umgebung in Kontakt stehen. Das Gehäuse kann aber auch ein innerhalb eines Außengehäuses liegendes inneres Gehäuse sein. Beispielsweise kann ein Inkubator mindestens eine als inneres Gehäuse dienende Kammer aufweisen, die durch mindestens eine Gehäusetüre bzw. Kammertüre verschließbar ist. In der Verschlussposition verschließt die Gehäusetüre das Gehäuseinnere vorzugsweise gasdicht, was insbesondere durch mindestens eine Dichtungseinrichtung der Gehäusetüre bzw. des Rahmens der Gehäuseöffnung gelingt. Die Erfindung betrifft aber grundsätzlich auch Laborschrankvorrichtungen mit einem Gehäuse, das das Gehäuseinnere gegenüber der Umgebung nicht vollständig abdichtet.

Die Laborschrankvorrichtung beinhaltet vorzugsweise eine Halteeinrichtung zum Halten des ersten und/oder des zweiten NTC-Temperatursensors im Abstand zu einer Innenwand und einer Bodenwand der Laborschrankvorrichtung, insbesondere im Abstand zu einer Innenwand und einer Bodenwand eines Gehäuses oder einer Kammer der Laborschrankvorrichtung. Insbesondere ist ein normaler Betriebszustand der Laborschrankvorrichtung vorgesehen, der auch als "erster Zustand" bezeichnet wird, während dem der erste NTC-Temperatursensor außerhalb der Flüssigkeit des Flüssigkeitsbehälters der Laborschrankvorrichtung angeordnet ist und der zweite NTC-Temperatursensor innerhalb der Flüssigkeit des Flüssigkeitsbehälters angeordnet ist. Insbesondere ist ein Fehler-Betriebszustand der Laborschrankvorrichtung vorgesehen, der auch als "zweiter Zustand" bezeichnet wird, während dem der erste NTC-Temperatursensor außerhalb der Flüssigkeit des Flüssigkeitsbehälters der Laborschrankvorrichtung angeordnet ist und der zweite NTC-Temperatursensor außerhalb der Flüssigkeit des Flüssigkeitsbehälters angeordnet ist. Die Laborschrankvorrichtung und/oder deren Steuereinrichtung und/oder deren Messeinrichtung sind dazu eingerichtet, durch die Messung des Vergleichswerts den Fehler-Betriebszustand vom normalen Betriebszustand zu unterscheiden und insbesondere über eine Benutzerschnittstelleneinrichtung eine Information über das Auftreten des Fehler-Betriebszustands an den Benutzer auszugeben.

Weitere bevorzugte Ausgestaltungen der erfindungsgemäßen Laborschrankvorrichtung lassen sich der Beschreibung der Ausführungsbeispiele gemäß der Figuren entnehmen.

Es zeigen:

Fig. 1a zeigt eine schematische Seitenansicht einer Laborschrankvorrichtung, die mit einer beispielhaften erfindungsgemäßen Füllstandsmessvorrichtung versehen ist, in einem ersten Zustand, in dem der zweite NTC-Temperatursensor innerhalb der Flüssigkeit des Flüssigkeitsbehälters der Laborschrankvorrichtung angeordnet ist.

Fig. 1b zeigt eine schematische Seitenansicht einer Laborschrankvorrichtung, die mit einer beispielhaften erfindungsgemäßen Füllstandsmessvorrichtung versehen ist, in einem zweiten Zustand, in dem der zweite NTC-Temperatursensor nicht mehr innerhalb der Flüssigkeit des Flüssigkeitsbehälters der Laborschrankvorrichtung angeordnet ist.

Fig. 2 zeigt eine Halteeinrichtung zum Halten des ersten und zweiten NTC-Temperatursensors einer beispielhaften erfindungsgemäßen Füllstandsmessvorrichtung, die insbesondere im Laborschrank gemäß Fig. 1a, 1b verwendbar ist.

Fig. 3 zeigt das Diagramm eines Vergleichswerts "Vout", der von der elektronischen Messeinrichtung einer beispielhaften erfindungsgemäßen Füllstandsmessvorrichtung fortlaufend ermittelt wurde, während zu einem bestimmten Zeitpunkt (nach 750 s) der zweite NTC-Temperatursensor aus der Flüssigkeit entfernt wurde.

Fig. 4 zeigt das Schaltungsprinzip einer spannungsgesteuerten Stromquelle, das bei der elektronischen Messeinrichtung einer beispielhaften erfindungsgemäßen Füllstandsmessvorrichtung angewandt wird, insbesondere bei der Schaltung in Fig. 5.

Fig. 5 zeigt den Schaltplan der Schaltung der elektronischen Messeinrichtung einer beispielhaften erfindungsgemäßen Füllstandsmessvorrichtung, die insbesondere bei der Füllstandsmessvorrichtung in Fig. 1a, 1b verwendet wird.

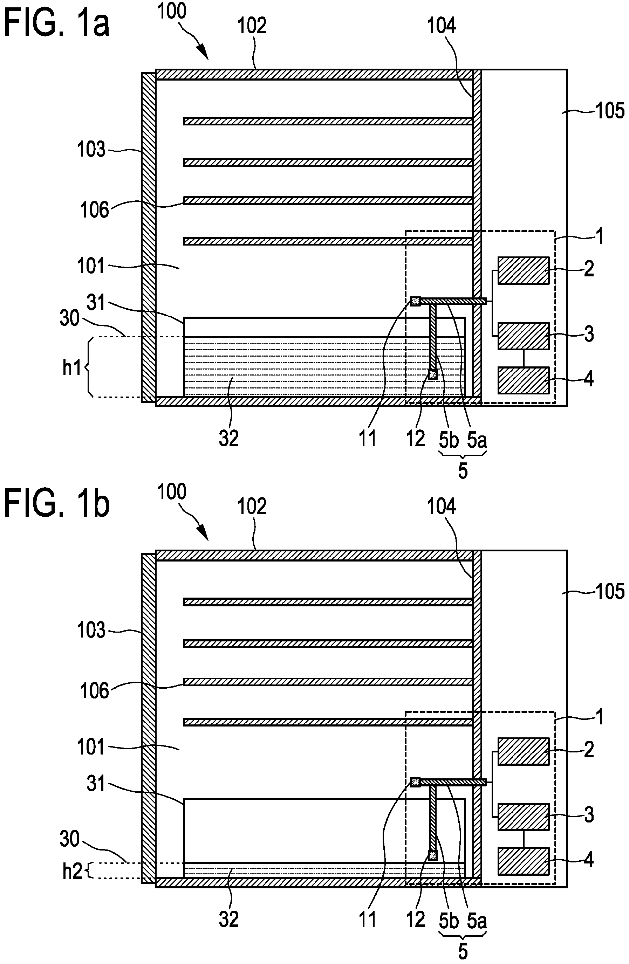

Fig. 1a zeigt eine Laborschrankvorrichtung 100, die mit einer Füllstandsmessvorrichtung versehen 1 ist, in einem ersten Zustand, in dem der zweite NTC-Temperatursensor 12 innerhalb der Flüssigkeit 32 des Flüssigkeitsbehälters 31 der Laborschrankvorrichtung 100 angeordnet ist. Fig. 1b zeigt dieselbe Laborschrankvorrichtung 100 in einem zweiten Zustand, in dem der zweite NTC-Temperatursensor 12, zum Beispiel wegen Verdunstung der Flüssigkeit, insbesondere Wasser, nicht mehr innerhalb der Flüssigkeit 32 des Flüssigkeitsbehälters 31 der Laborschrankvorrichtung angeordnet ist.

Die Laborschrankvorrichtung 100 ist vorliegend ein CO2-Inkubator für das Wachstum lebender Zellkulturen. Der Inkubator 100 ist vereinfacht dargestellt. Er weist ein Gehäuse 102 auf, das den auf 37°C temperaturgeregelten (temperierten) Innenraum 101 thermisch von der Umgebung isoliert. Die Gehäuseöffnung ist von einer Gehäusetüre 103 verschlossen und gewährt Zugang zum Innenraum 101. Im Innenraum befinden sich gelochte Einlegeböden 106, am Boden des Innenraums findet sich der als Wasserwanne ausgebildete Flüssigkeitsbehälter 31, in dem die Flüssigkeit, der Gravitation folgend, horizontal angeordnet und parallel zur Bodenplatte des Gehäuses angeordnet ist. Die Rückwand 104 des Gehäuses ist senkrecht zur Bodenplatte und somit vertikal angeordnet. An der Innenseite dieser Rückwand ist im Innenraum 101 des Inkubators die Halteeinrichtung 5 des Inkubators montiert.

Die Halteeinrichtung 5 des Inkubators weist einen horizontal verlaufenden ersten Haltearm 5a auf, der an der Rückwand 104 befestigt ist. An der vertikal nach unten weisenden Seite des ersten Haltearms 5a ist der zweite Haltearm 5b befestigt. Der erste NTC-Temperatursensor 11 ist am Ende des ersten Haltearms 5a befestigt, der zweite NTC-Temperatursensor 12 ist am Ende des zweiten Haltearms 5b befestigt. Durch diese Anordnung liegt der erste NTC-Temperatursensor 11 immer oberhalb der Flüssigkeitsoberfläche 30 und somit immer außerhalb der Flüssigkeit. Der erste NTC-Temperatursensor 11 grenzt grundsätzlich immer an die gasförmige Atmosphäre (Luft mit geregelter $CO_2$, $H_2O$ Zusammensetzung) des Innenraums 101. Der zweite NTC-Temperatursensor 12 reicht im ersten Zustand eines ausreichend befüllten Wasserbehälters 31, wie in Fig. 1a dargestellt, immer in die Flüssigkeit hinein. Im (Fehler-) Fall bzw. im zweiten Zustand eines nicht ausreichend befüllten Wasserbehälters 31, wie in Fig. 1b dargestellt, steht der zweite NTC-Temperatursensor 12 nicht mehr im Kontakt mit der Flüssigkeit der Flüssigkeitsoberfläche 30. Im ersten Zustand weist der Füllstand, gemessen vom Boden des Wasserbehälters 31, die Höhe h1 auf. Im zweiten Zustand weist der Füllstand, gemessen vom Boden des Wasserbehälters 31, die Höhe h2 auf.

Die Füllstandsmessvorrichtung ist in Fig. 1a und 1b mit einem gestrichelten Rechteck mit dem Bezugszeichen 1 umrahmt. Der erste Haltearm 5a ragt durch einen Port in der Rückwand 104 des Gehäuses in den Steuerungsraum 105 des Inkubators, in dem die Stromversorgung 2, die elektronische Messeinrichtung 3 und die Auswertungseinrichtung 4 angeordnet sind.

Die Füllstandsmessvorrichtung 1 dient der Messung des Füllstands 30 im Flüssigkeitsbehälter 31 und weist auf: einen ersten NTC-Temperatursensor 11, der außerhalb der Flüssigkeit 32 des Flüssigkeitsbehälters 31 anordenbar ist bzw. angeordnet ist, einen zweiten NTC-Temperatursensor 12, der innerhalb der Flüssigkeit 32 des Flüssigkeitsbehälters 31 anordenbar ist bzw. im Normalfall angeordnet ist. Die Füllstandsmessvorrichtung 1 weist eine Stromversorgungseinrichtung 2 auf, von der der erste NTC-Temperatursensor 11 und der zweite NTC-Temperatursensor 12 so mit Strom versorgt werden, dass der erste NTC-Temperatursensor 11 und der zweite NTC-Temperatursensor 12 jeweils eine Übertemperatur T_NTC aufweisen, die größer ist als die Temperatur T_innen im Innenraum 101 der Laborschrankvorrichtung. Beispielsweise ist T_innen = 37 ° C, dann ist T_NTC vorzugsweise um 4 bis 15 °C höher, insbesondere vorliegend zwischen 40 und 50 °C.

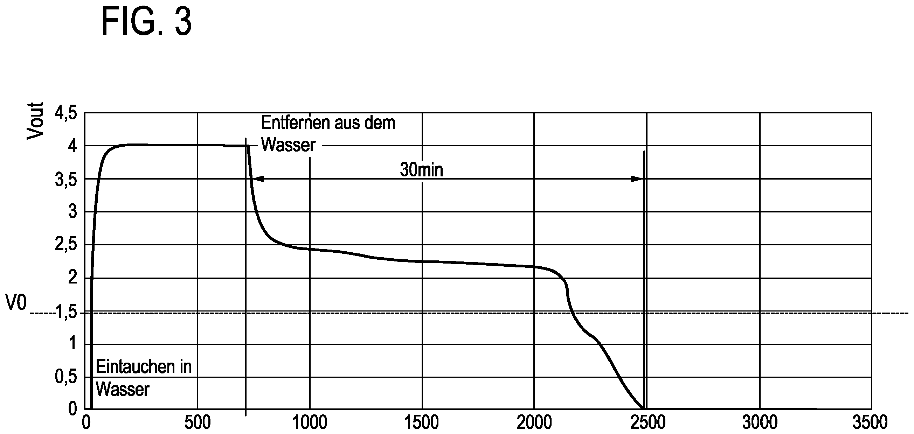

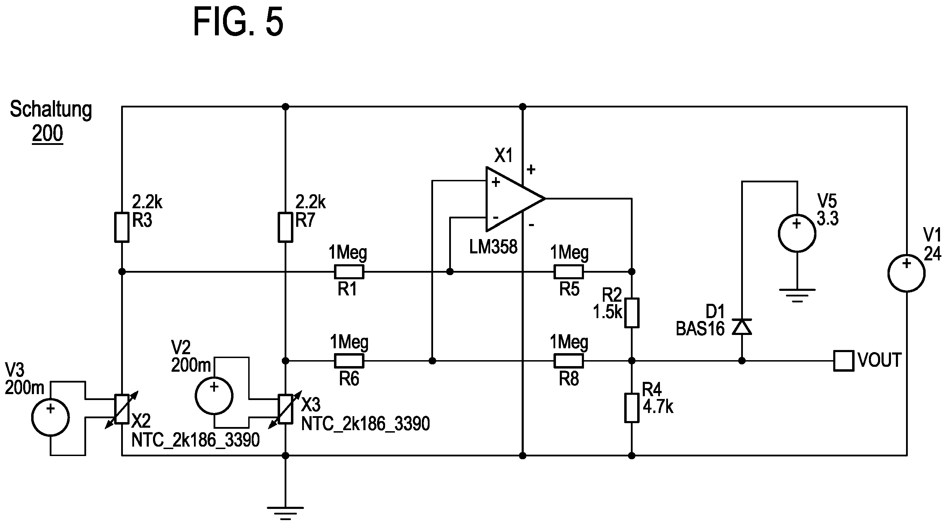

Die elektronische Messeinrichtung 3 ist dazu eingerichtet, eine von der Temperatur des ersten NTC-Temperatursensors 11 beeinflusste erste elektrische Größe des ersten NTC-Temperatursensors 11, z.B. eine Spannungsänderung am ersten NTC-Temperatursensor, zu erfassen und eine von der Temperatur des zweiten NTC-Temperatursensors 12 beeinflusste zweite elektrische Größe des zweiten NTC-Temperatursensors 12, z.B. eine Spannungsänderung am zweiten NTC-Temperatursensor, zu erfassen, und durch fortlaufenden Vergleich, also einen in kurzen Zeitabständen zwischen z.B. 50 ms und 1 min durchgeführten Vergleich, der ersten und zweiten elektrischen Größe einen Vergleichswert, z.B. "Vout" in den Figuren 3 und 5, zu ermitteln.

Die Auswertungseinrichtung 4 weist eine Datenspeichereinrichtung und eine Datenverarbeitungseinrichtung (jeweils nicht gezeigt) auf, die dazu eingerichtet ist, den zweiten Vergleichswert vom ersten Vergleichswert zu unterscheiden, indem der fortlaufend gemessen Vergleichswert mit einem Referenzwert verglichen wird, der in der Datenspeichereinrichtung gespeichert ist.

Die elektronische Messeinrichtung 3 ist dazu eingerichtet ist, einen ersten Vergleichswert zu ermitteln, wenn der Füllstand 30 im Flüssigkeitsbehälter oberhalb des zweiten NTC-Temperatursensors 12 steht, wie in Fig. 1a dargestellt, und einen vom ersten Vergleichswert abweichenden zweiten Vergleichswert zu erfassen, wenn der Füllstand 30 im Flüssigkeitsbehälter unter den zweiten NTC-Temperatursensor 12 gesunken ist. Der erste Vergleichswert Vout liegt in Fig. 3 zum Zeitpunkt t=0 bei ca. Vout=4 V, der zweite Vergleichswert Vout liegt zum Zeitpunkt t=2500 s bei Vout=0 V. Die Auswertungseinrichtung 4 entscheidet anhand eines in der Datenspeichereinrichtung der Auswertungseinrichtung 4 gespeicherten Referenzwertes V0, ob der minimal zulässige Füllstand des Flüssigkeitsbehälters unterschritten wurde. Der Wert V0 ist ein Schwellwert und liegt vorliegend bei ca. V0=1,5 V, der hier zum Zeitpunkt t= 2200 s erreicht wird, im Diagramm der Fig. 3 ist das ca. 1450 s, nachdem der zweite NTC-Temperatursensor 12 zu Testzwecken aus dem Wasserbad 32 entfernt wurde, in das er zum Zeitpunkt t=0 platziert worden war.

In Fig. 3 ist erkennbar, dass der zweite NTC-Temperatursensor 12 nach dem Einsetzen ins Wasser eine Zeit von ca. 120 s benötigt, bis ein stationäres Gleichgewicht der Temperatur des NTC-Sensors erreicht ist, dass sich aus der Leistungszufuhr der Stromversorgung des Sensors und der Wärmeabgabe über seine Sensorkontaktfläche ergibt. Der Kurvenverlauf zwischen t=750s und t=2500 s ergibt sich daraus, dass die Sensorkontaktfläche eine Zeit lang noch von einem Meniskus der Flüssigkeit 32 benetzt ist, bevor die Flüssigkeit nicht mehr in Kontakt mit der Sensorkontaktfläche steht und die differentielle Messung zwischen dem ersten und zweiten NTC-Temperatursensor eine Vergleichsspannung von 0V ergibt.

Die Auswertungseinrichtung 4 weist eine Datenspeichereinrichtung und eine Datenverarbeitungseinrichtung auf, die hier jeweils nicht gezeigt sind, die dazu eingerichtet sind, den zweiten Vergleichswert vom ersten Vergleichswert zu unterscheiden, indem der fortlaufend gemessen Vergleichswert mit einem Referenzwert verglichen wird, der in der Datenspeichereinrichtung gespeichert ist.

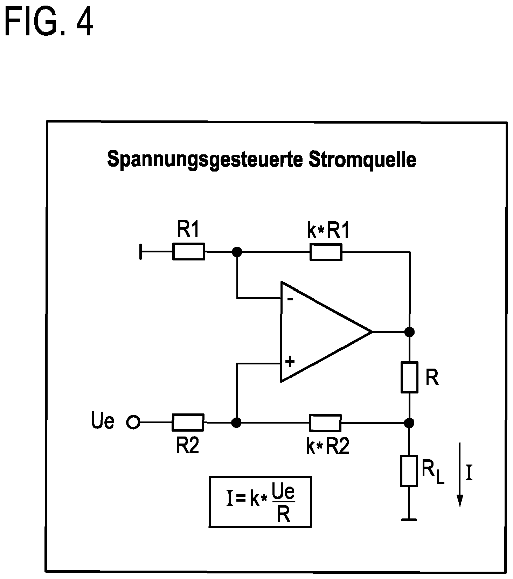

Die elektronische Messeinrichtung 3 ist so eingerichtet, dass der erste und der zweite NTC-Temperatursensor in einer Brückenschaltung 200 angeordnet sind, siehe Fig. 5. Als der erste und der zweite Vergleichswert wird jeweils die Brückenspannung Vout der Brückenschaltung 200 verwendet. Die elektronische Messeinrichtung 3 weist eine spannungsgesteuerte Stromquelle als elektronische Schaltung 400 auf (siehe Fig. 4), deren Eingangsspannung die Brückenspannung ist. Der Ausgangsstrom wird verwendet, um über einen Lastwiderstand eine massebezogene Ausgangsspannung zu erzeugen, die an einen ADC-Eingang geht.

In Fig. 5 ist der erste NTC-Temperatursensor mit "X2, NTC_2k186_3390" bezeichnet, der zweite NTC-Temperatursensor mit "X3, NTC_2k186_3390". Die Eingangsspannung ist die Brückenspannnung der NTC-Temperatursensoren (differentielle Messung). Der Ausgangsstrom des Operationsverstärkers wird verwendet, um mit R4 eine massebezogene Ausgangsspannung zu erzeugen, die an einen ADC-Eingang geht. Die Empfindlichkeit (Verstärkung) der Schaltung wird mit R2 eingestellt. D1 begrenzt die ADC-Eingangsspannung. Es gilt:

$$Vout = R4 * (U\_NTC\_x3 - U\_NTC\_x2 ) / R2$$

R3/R7 stellen den Meßstrom der NTC's ein, damit es zu einer nennenswerten Erwärmung kommt.

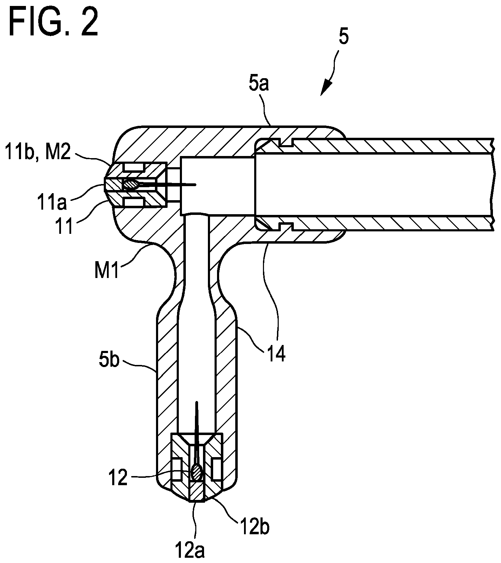

Die Füllstandsmessvorrichtung weist die Halteeinrichtung 5 zum Halten des ersten und/oder des zweiten NTC-Temperatursensors im Abstand zur Innenwand 104 der Laborschrankvorrichtung auf, die anhand der Figur 2 erläutert wird. Die Halteeinrichtung 5 weist einen an einer Innenwand der Laborschrankvorrichtung 100 befestigbaren Haltearm 5a auf, der den ersten NTC-Temperatursensor 11 trägt und an dem ein elastisch verformbarerer zweiter Haltearm 5b angebracht ist, der für die vertikal nach unten gerichtete Anordnung vorgesehen ist und der den zweiten NTC-Temperatursensor 12 trägt. Die Halteeinrichtung 5 weist eine Kapseleinrichtung 14 aus einem ersten Material M1 auf und mindestens eine Sensorkontaktfläche 11a, 12a des ersten und/oder des zweiten NTC-Temperatursensors 11, 12 aus einem zweiten Material M2, so dass die Kapseleinrichtung 14 die beiden Sensorkontaktfläche 11a, 12a einfasst. Die Kapseleinrichtung 14 und die beiden Sensorkontaktflächen 11a, 12a begrenzen den ersten und den zweiten NTC-Temperatursensor 11, 12 nach außen. Das zweite Material M2 weist eine höhere Wärmeleitfähigkeit auf als das erste Material M1. Das erste Material M1 ist ein Kunststoff, insbesondere Gummi oder ein Elastomer, und das zweite Material M2 ist hier Edelstahl. Auf diese Weise wurde das technische Problem gelöst, das in der Auswahl der NTC-Temperatursensoren und der mechanischen Ausführung der Kapseleinrichtung 14 der NTC-Temperatursensoren liegt. Da der NTC-Temperatursensor die Wärmeenergie an die Kapseleinrichtung abgibt, muss der NTC-Temperatursensor groß genug sein und die Kapseleinrichtung so konstruiert sein, dass zu einer nennenswerten Erwärmung des NTC-Temperatursensors auf die Übertemperatur T_NTC kommt, hier ca. 50 °C. Für die Fühler kommen insbesondere niederohmige Typen (R25 ca. 1k -2k) in Frage, weil sonst die bevorzugte Versorgungsspannung (24V) nicht ausreicht, um den notwendigen Strom bereitzustellen. Da die NTC-Temperatursensoren für 180°C geeignet sein sollen, werden glasspassivierte Typen bevorzugt.

Der erste NTC-Temperatursensor 11 und dessen Sensorkontaktfläche 11a und der zweite NTC-Temperatursensor 12 und dessen Sensorkontaktfläche 12a sind jeweils von einer zylinderartigen Fassung 11b, 12b eingefasst sind, so dass die Kapseleinrichtung 14, die Fassung 11b, 12b des ersten und des zweiten NTC-Temperatursensors 11, 12 und die mindestens eine Sensorkontaktfläche 11a, 12a den ersten und den zweiten NTC-Temperatursensor 11, 12 nach außen begrenzen. Die Fassung 11b, 12b besteht aus einem dritten Material M3, hier Polyetheretherketon (PEEK) und ist damit temperaturbeständig und chemisch inert. Die Halteeinrichtung 5, der erste NTC-Temperatursensor 11 und der zweite NTC-Temperatursensor 12 bestehen aus Materialien, die Betriebstemperaturen des Innenraums der Laborschrankvorrichtung von bis zu 180° C widerstehen.

**Patentansprüche**

1. Füllstandsmessvorrichtung (1) zur Messung eines Füllstands (30) in einem Flüssigkeitsbehälter (31) im Innenraum (101) einer Laborschrankvorrichtung (100), aufweisend

   einen ersten NTC-Temperatursensor (11), der außerhalb der Flüssigkeit (32) des Flüssigkeitsbehälters (31) anordenbar ist,
   einen zweiten NTC-Temperatursensor (12), der innerhalb der Flüssigkeit (32) des Flüssigkeitsbehälters (31) anordenbar ist,
   eine Stromversorgungseinrichtung (2), von der der erste NTC-Temperatursensor (11) und der zweite NTC-Temperatursensor (12) so mit Strom versorgt werden, dass der erste NTC-Temperatursensor (11) und der zweite NTC-Temperatursensor (12) jeweils eine Übertemperatur (T_NTC) aufweisen, die größer ist als die Temperatur (Tinnen) im Innenraum (101) der Laborschrankvorrichtung, und
   eine elektronische Messeinrichtung (3), die dazu eingerichtet ist, eine von der Temperatur des ersten NTC-Temperatursensors (11) beeinflusste erste elektrische Größe des ersten NTC-Temperatursensors (11) zu erfassen und eine von der Temperatur des zweiten NTC-Temperatursensors (12) beeinflusste zweite elektrische Größe des zweiten NTC-Temperatursensors (12) zu erfassen, und durch fortlaufenden Vergleich der ersten und zweiten elektrischen Größe einen Vergleichswert zu ermitteln,
   **gekennzeichnet durch**
   eine Halteeinrichtung (5) zum Halten des ersten und des zweiten NTC-Temperatursensors im Abstand zu einer Innenwand und einer Bodenwand der Laborschrankvorrichtung (100), wobei die Halteeinrichtung (5) einen an der Innenwand der Laborschrankvorrichtung (100) befestigbaren Haltearm (5a) aufweist, der den ersten NTC-Temperatursensor (11) trägt und an dem ein elastisch verformbarerer zweiter Haltearm (5b) angebracht ist, der für eine vertikal nach unten gerichtete Anordnung vorgesehen ist und der den zweiten NTC-Temperatursensor (12) trägt.

2. Füllstandsmessvorrichtung gemäß Anspruch 1, **dadurch gekennzeichnet, dass** die elektronische Messeinrichtung dazu eingerichtet ist, einen ersten Vergleichswert zu ermitteln, wenn der Füllstand (30) im Flüssigkeitsbehälter oberhalb des zweiten NTC-Temperatursensors (12) steht, und einen vom ersten Vergleichswert abweichenden zweiten Vergleichswert zu erfassen, wenn der Füllstand (30) im Flüssigkeitsbehälter unter den zweiten NTC-Temperatursensor (12) gesunken ist.

3. Füllstandsmessvorrichtung gemäß Anspruch 1 oder 2, die eine Auswertungseinrichtung (4) mit einer Datenspeichereinrichtung und einer Datenverarbeitungseinrichtung aufweist, die dazu eingerichtet ist, einen zweiten Vergleichswert von einem ersten Vergleichswert zu unterscheiden, indem der fortlaufend gemessen Vergleichswert mit einem Referenzwert verglichen wird, der in der Datenspeichereinrichtung gespeichert ist.

4. Füllstandsmessvorrichtung gemäß einem der vorangehenden Ansprüche, **dadurch gekennzeichnet, dass** der Vergleichswert ein Differenzwert ist, und dass die elektronische Messeinrichtung (3) dazu eingerichtet ist, eine Differenz der ersten elektrischen Größe des ersten NTC-Temperatursensors (11) und der zweiten elektrischen Größe des zweiten NTC-Temperatursensors (12) zu bilden, und durch fortlaufende Differenzbildung der ersten und zweiten elektrischen Größe den Vergleichswert zu ermitteln.

5. Füllstandsmessvorrichtung gemäß einem der vorangehenden Ansprüche, **dadurch gekennzeichnet, dass** die elektronische Messeinrichtung (3) so eingerichtet ist, dass der erste und der zweite NTC-Temperatursensor in einer Brückenschaltung (200) angeordnet sind und dass als der erste und der zweite Vergleichswert jeweils die Brückenspannung der Brückenschaltung (200) verwendet wird.

6. Füllstandsmessvorrichtung gemäß Anspruch 5, **dadurch gekennzeichnet, dass** die elektronische Messeinrichtung (3) eine spannungsgesteuerte Stromquelle als elektronische Schaltung (400) aufweist, deren Eingangsspannung die Brückenspannung ist.

7. Füllstandsmessvorrichtung gemäß einem der vorangehenden Ansprüche, **dadurch gekennzeichnet, dass** die Halteeinrichtung (5) eine Kapseleinrichtung (14) aus einem ersten Material (M1) aufweist und dass mindestens eine Sensorkontaktfläche (11a, 12a) des ersten und/oder des zweiten NTC-Temperatursensors (11, 12) aus einem zweiten Material M2) vorgesehen sind, so dass die Kapseleinrichtung (14) die mindestens eine Sensorkontaktfläche (11a, 12a) einfasst und die Kapseleinrichtung (14) und die mindestens eine Sensorkontaktfläche (11a, 12a) den ersten und/oder den zweiten NTC-Temperatursensor (11, 12) nach außen begrenzen.

8. Füllstandsmessvorrichtung gemäß Anspruch 7, **dadurch gekennzeichnet, dass** das zweite Material (M2) eine höhere Wärmeleitfähigkeit aufweist als das erste Material (M1).

9. Füllstandsmessvorrichtung gemäß Anspruch 8, **dadurch gekennzeichnet, dass** das erste Material (M1) ein Kunststoff ist und das zweite Material (M2) ein Metall, insbesondere Edelstahl, ist.

10. Füllstandsmessvorrichtung gemäß einem der Ansprüche 7 bis 9, **dadurch gekennzeichnet, dass** der erste NTC-Temperatursensor (11) und dessen Sensorkontaktfläche (11a) und/oder der zweite NTC-Temperatursensor (12) und dessen Sensorkontaktfläche (12a) jeweils von einer Fassung (11b, 12b) eingefasst sind, so dass die Kapseleinrichtung (14), die Fassung (11b, 12b) des ersten und/oder des zweiten NTC-Temperatursensors (11, 12) unmittelbar und die mindestens eine Sensorkontaktfläche (11a, 12a) des ersten und/oder des zweiten NTC-Temperatursensor (11, 12) mittelbar nach außen begrenzen.

11. Füllstandsmessvorrichtung gemäß Anspruch 9, **dadurch gekennzeichnet, dass** die Fassung (11b, 12b) aus einem dritten Material (M3), insbesondere Polyetheretherketon (PEEK) besteht.

12. Füllstandsmessvorrichtung gemäß einem der vorangehenden Ansprüche, **dadurch gekennzeichnet, dass** die Halteeinrichtung (5), der erste NTC-Temperatursensor (11) und der zweite NTC-Temperatursensor (12) aus Materialien bestehen, die Betriebstemperaturen des Innenraums der Laborschrankvorrichtung von bis zu 180° C widerstehen.

13. Laborschrankvorrichtung (100) zum Lagern von Laborproben, insbesondere Temperierschrank, mit einem Flüssigkeitsbehälter (31) im Innenraum (101) der Laborschrankvorrichtung (100) und einer Füllstandsmessvorrichtung (1) gemäß einem der Ansprüche 1 bis 12 zur Messung eines Füllstands (30) in dem Flüssigkeitsbehälter (31).

14. Verwendung der Füllstandsmessvorrichtung (1) gemäß einem der Ansprüche 1 bis 12 zur Messung des Füllstands des Flüssigkeitsbehälters einer Luftbefeuchtungseinrichtung eines Inkubators.

**Claims**

1. Level measuring device (1) for measuring a level (30) in a liquid container (31) in the interior (101) of a laboratory cabinet device (100), comprising

    a first NTC temperature sensor (11) arrangeable outside the liquid (32) of the liquid container (31)
    a second NTC temperature sensor (12) arrangeable inside the liquid (32) of the liquid container (31)
    a power supply device (2), of which the first NTC temperature sensor (11) and the second NTC temperature sensor (12) each comprise an excess temperature (T_NTC) which is greater than the temperature (T_inside) in the interior (101) of the laboratory cabinet device, and
    an electronic measuring device (3) which is set up to detect a first electrical variable of the first NTC temperature sensor (11) which is influenced by the temperature of the first NTC temperature sensor (11), and to detect a second electrical variable of the second NTC temperature sensor (12) which is influenced by the temperature of the second NTC temperature sensor (12), and to determine a comparison value by continuous comparison of the first and second electrical variables,
    **characterized by**
    a holding device (5) for holding the first and the second NTC temperature sensor at a distance from an inner wall and a bottom wall of the laboratory cabinet device (100), the holding device (5) having a holding arm (5a) which can be fastened to the inner wall of the laboratory cabinet device (100) which supports the first NTC temperature sensor (11) and to which an elastically deformable second holding arm (5b) is attached, which is provided for a vertically downwardly directed arrangement and which supports the second NTC temperature sensor (12).

2. Level measuring device according to claim 1, **characterized in that** the electronic measuring device is arranged to determine a first comparison value when the level (30) in the liquid container is above the second NTC temperature sensor (12) and to detect a second comparison value deviating from the first comparison value when the level (30) in the liquid container has fallen below the second NTC temperature sensor (12).

3. Level measuring device according to claim 1 or 2, comprising evaluation means (4) with data storage means and data processing means adapted to distinguish a second comparison value from a first comparison value by comparing the continuously measured comparison value with a reference value stored in the data storage means.

4. Level measuring device according to one of the preceding claims, **characterized in that** the comparison value is a differential value, and **in that** the electronic measuring device (3) is set up to form a difference between the first electrical quantity of the first NTC temperature sensor (11) and the second electrical quantity of the second NTC temperature sensor (12), and to determine the comparison value by continuously forming the difference between the first and second electrical quantities.

5. Level measuring device according to one of the preceding claims, **characterized in that** the electronic measuring device (3) is set up in such a way that the first and the second NTC temperature sensor are arranged in a bridge circuit (200), and **in that** the bridge voltage of the bridge circuit (200) is used as the first and the second comparison value, respectively.

6. Level measuring device according to claim 5, **characterized in that** the electronic measuring device (3) comprises a voltage-controlled current source as electronic circuit (400) whose input voltage is the bridge voltage.

7. Level measuring device according to one of the preceding claims, **characterized in that** the holding device (5) comprises a capsule device (14) made of a first material (M1) and that at least one sensor contact surface (11a, 12a) of the first and/or the second NTC temperature sensor (11, 12) are provided of a second material (M2), so that the capsule device (14) encloses the at least one sensor contact surface (11a, 12a) and the capsule device (14) and the at least one sensor contact surface (11a, 12a) delimit the first and/or the second NTC temperature sensor (11, 12) to the outside.

8. Level measuring device according to claim 7, **characterized in that** the second material (M2) comprises a higher thermal conductivity than the first material (M1).

9. Level measuring device according to claim 8, **characterized in that** the first material (M1) is a plastic and the second material (M2) is a metal, in particular stainless steel.

10. Level measuring device according to one of claims 7 to 9, **characterized in that** the first NTC temperature sensor (11) and its sensor contact surface (11a) and/or the second NTC temperature sensor (12) and its sensor contact surface (12a) are each enclosed by a socket (11b, 12b), so that the capsule device (14), the socket (11b, 12b) of the first and/or of the second NTC temperature sensor (11, 12) directly and the at least one sensor contact surface (11a, 12a) of the first and/or of the second NTC temperature sensor (11, 12) indirectly delimit towards the outside.

11. Level measuring device according to claim 9, **characterized in that** the socket (11b, 12b) consists of a third material (M3), in particular polyetheretherketone (PEEK).

12. Level measuring device according to one of the preceding claims, **characterized in that** the holding device (5), the first NTC temperature sensor (11) and the second NTC temperature sensor (12) are made of materials which withstand operating temperatures of the interior of the laboratory cabinet device of up to 180 °C.

13. Laboratory cabinet device (100) for storing laboratory samples, in particular temperature control cabinet, having a liquid container (31) in the interior (101) of the laboratory cabinet device (100) and a level measuring device (1) according to one of claims 1 to 12 for measuring a level (30) in the liquid container (31).

14. Use of the level measuring device (1) according to one of claims 1 to 12 for measuring the level of the liquid container of an air humidification device of an incubator.

**Revendications**

1. Dispositif de mesure de niveau de remplissage (1) destiné à mesurer un niveau de remplissage (30) dans un récipient à liquide (31) dans l'espace intérieur (101) d'un dispositif d'armoire de laboratoire (100), présentant

   un premier capteur de température NTC (11) qui peut être agencé à l'extérieur du liquide (32) du récipient à liquide (31),
   un second capteur de température NTC (12) qui peut être agencé à l'intérieur du liquide (32) du récipient à liquide (31),
   un équipement d'alimentation en courant (2) par lequel le premier capteur de température NTC (11) et le second capteur de température NTC (12) peuvent ainsi être alimentés en courant, en ce que le premier capteur de température NTC (11) et le second capteur de température NTC (12) présentent respectivement une surtempérature (T_NTC) qui est supérieure à la température (T_innen) dans l'espace intérieur (101) du dispositif d'armoire de laboratoire, et
   un équipement de mesure (3) électronique qui est conçu pour saisir une première grandeur électrique du premier capteur de température NTC (11), influencée par la température du premier capteur de température NTC (11), et pour saisir une seconde grandeur électrique du second capteur de température NTC (12), influencée par la température du second capteur de température NTC (12), et pour établir une valeur de comparaison par comparaison en continu des première et seconde grandeurs électriques,
   **caractérisé par**
   un équipement de retenue (5) pour retenir le premier et le second capteurs de température NTC à distance d'une paroi intérieure et d'une paroi inférieure du dispositif d'armoire de laboratoire (100), dans lequel l'équipement de retenue (5) présente un bras de retenue (5a) pouvant être fixé contre la paroi intérieure du dispositif d'armoire de laboratoire (100), lequel bras de retenue porte le premier capteur de température NTC (11) et contre lequel est monté un second bras de retenue (5b) élastiquement déformable, qui est prévu pour un agencement orienté verticalement vers le bas et qui porte le second capteur de température NTC (12).

2. Dispositif de mesure de niveau de remplissage selon la revendication 1, **caractérisé en ce que** l'équipement de mesure électronique est conçu pour établir une première valeur de comparaison lorsque le niveau de remplissage (30) dans le récipient à liquide se situe au-dessus du second capteur de température NTC (12) et pour saisir une seconde valeur de comparaison s'écartant de la première valeur de comparaison lorsque le niveau de remplissage (30) dans le récipient à liquide est tombé en-dessous du second capteur de température NTC (12).

3. Dispositif de mesure de niveau de remplissage selon la revendication 1 ou 2, qui présente un équipement d'analyse (4) avec un équipement de mémoire de données et un équipement de traitement de données, lequel équipement d'analyse est conçu pour différencier une seconde valeur de comparaison d'une première valeur de comparaison en ce que la valeur de comparaison mesurée en continu est comparée à une valeur de référence qui est mémorisée dans l'équipement de mémoire de données.

4. Dispositif de mesure de niveau de remplissage selon l'une des revendications précédentes, **caractérisé en ce que** la valeur de comparaison est une valeur de différence, et **en ce que** l'équipement de mesure (3) électronique est conçu pour former une différence entre la première grandeur électrique du premier capteur de température NTC (11) et la seconde grandeur électrique du second capteur de température NTC (12), et pour établir la valeur de comparaison par formation de différence en continu des première et seconde grandeurs électriques.

5. Dispositif de mesure de niveau de remplissage selon l'une des revendications précédentes, **caractérisé en ce que** l'équipement de mesure (3) électronique est conçu de sorte que le premier et le second capteurs de température NTC soient agencés dans un montage en pont (200) et **en ce que**, en tant que première et seconde valeur de comparaison, soit respectivement utilisée la tension de pont du montage en pont (200).

6. Dispositif de mesure de niveau de remplissage selon la revendication 5, **caractérisé en ce que** l'équipement de mesure (3) électronique présente, en tant que montage électronique (400), une source de courant commandée par une tension, dont la tension d'entrée est la tension de pont.

7. Dispositif de mesure de niveau de remplissage selon l'une des revendications précédentes, **caractérisé en ce que** l'équipement de retenue (5) présente un équipement de capsule (14) fabriqué dans un premier matériau (M1) et **en ce qu'**au moins une surface de contact de capteur (11a, 12a) du premier et/ou du second capteur de température NTC (11, 12) sont prévus dans un deuxième matériau (M2), de sorte que l'équipement de capsule (14) entoure la au moins une surface de contact de capteur (11a, 12a), et l'équipement de capsule (14) et la au moins une surface de contact de capteur (11a, 12a) délimitent vers l'extérieur le premier et/ou le second capteur de température NTC (11, 12).

8. Dispositif de mesure de niveau de remplissage selon la revendication 7, **caractérisé en ce que** le deuxième matériau (M2) présente une conductivité thermique supérieure à celle du premier matériau (M1).

9. Dispositif de mesure de niveau de remplissage selon la revendication 8, **caractérisé en ce que** le premier matériau (M1) est une matière plastique et le deuxième matériau (M2) est un métal, en particulier un acier spécial.

10. Dispositif de mesure de niveau de remplissage selon l'une des revendications 7 à 9, **caractérisé en ce que** le premier capteur de température NTC (11) et la surface de contact de capteur (11a) de celui-ci et/ou le second capteur de température NTC (12) et la surface de contact de capteur (12a) sont respectivement entourés par une douille (11b, 12b), de sorte que l'équipement de capsule (14) délimite vers l'extérieur directement la douille (11b, 12b) du premier et/ou du second capteur de température NTC (11, 12) et indirectement la au moins une surface de contact de capteur (11a, 12a) du premier et/ou du second capteur de température NTC (11, 12).

11. Dispositif de mesure de niveau de remplissage selon la revendication 9, **caractérisé en ce que** la douille (11b, 12b) consiste en un troisième matériau (M3), en particulier une polyétheréthercétone (PEEK).

12. Dispositif de mesure de niveau de remplissage selon l'une des revendications précédentes, **caractérisé en ce que** l'équipement de retenue (5), le premier capteur de température NTC (11) et le second capteur de température NTC (12) consistent en des matériaux qui résistent à des températures de service de l'espace intérieur du dispositif d'armoire de laboratoire allant jusqu'à 180°C.

13. Dispositif d'armoire de laboratoire (100) destiné à stocker des échantillons de laboratoire, en particulier une armoire de mise en température, avec un récipient à liquide (31) dans l'espace intérieur (101) du dispositif d'armoire de laboratoire (100) et un dispositif de mesure de niveau de remplissage (1) selon l'une des revendications 1 à 12 pour mesurer un niveau de remplissage (30) dans le récipient à liquide (31).

14. Utilisation du dispositif de mesure de niveau de remplissage (1) selon l'une des revendications 1 à 12 pour mesurer le niveau de remplissage du récipient à liquide d'un équipement d'humidification d'un incubateur.

FIG. 1a

FIG. 1b

FIG. 2

FIG. 3

# FIG. 4

**Spannungsgesteuerte Stromquelle**

$$I = k * \frac{Ue}{R}$$

FIG. 5

EP 3 765 589 B1

**EP 3 765 589 B1**

**IN DER BESCHREIBUNG AUFGEFÜHRTE DOKUMENTE**

**In der Beschreibung aufgeführte Patentdokumente**

- EP 2067849 B1

- JP 2002188977 A